Europäisches Patentamt

European Patent Office

Office européen des brevets

(11) Veröffentlichungsnummer: **0 476 495 A1**

# EUROPÄISCHE PATENTANMELDUNG

(21) Anmeldenummer: **91115331.0**

(22) Anmeldetag: **11.09.91**

(51) Int. Cl.5: **A61B 8/14**, G10K 11/00, G01S 7/52

(30) Priorität: **20.09.90 DE 4029829**

(43) Veröffentlichungstag der Anmeldung:
**25.03.92 Patentblatt 92/13**

(84) Benannte Vertragsstaaten:
**DE FR GB IT**

(71) Anmelder: **DORNIER MEDIZINTECHNIK GMBH**
**Postfach 1128**
**W-8000 München(DE)**

(72) Erfinder: **Hesse, Alex, Dr.**

Guntherstrasse 15
**W-8000 München 19(DE)**
Erfinder: **Nuber, Bernd, Ing.**
Karlstrasse 120
**W-8000 München 2(DE)**
Erfinder: **Hornung, Bernhard**
St. Gilgen 8a
**W-8031 Gilching(DE)**

(74) Vertreter: **Kasseckert, Rainer**
**DORNIER GMBH Kleeweg 3**
**W-7990 Friedrichshafen 1(DE)**

(54) **Dreidimensionales Darstellen von Ultraschall-Bildern.**

(57) Vorrichtung zum dreidimensionalen Darstellen von Ultraschallbildern, insbesondere eines Körperorgans, mit einem Schallkopf (**SK**), der in einem handhabbaren Gehäuse (**G**) angeordnet ist, wobei ein Transducer-Array (**TA**) von einem Schrittmotor (**SM**) im vorgegebenen Winkelbereich positioniert wird und die Stellung des Transducer-Arrays (**TA**) registriert wird.

Fig. 2

Die Erfindung betrifft eine Vorrichtung zum dreidimensionalen Darstellen von Ultraschall-Bildern, insbesondere eines Körperorgans, nach dem Oberbegriff des Anspruchs 1.

Die dreidimensionale Darstellung von Organen, Tumoren und ganzen Körperteilen was bisher der Kernspintomographie und Computertomographie vorbehalten. Aus den entsprechenden Schnittbildern, die mit Hilfe dieser beiden Verfahren von einem Körper gewonnen werden, wird eine dreidimensionale Darstellung errechnet. Die dabei erzeugten äquidistanten Schnittbilder liegen immer parallel zueinander. Durch die parallele Folge der zweidimensionalen Schnitte wird das untersuchte Gewebe räumlich abgescannt. Mit Hilfe der digitalen Bildverarbeitung kann die gewonnene Information räumlich rekonstruiert und dargestellt werden.

Es wurde bereits eine Vorrichtung zum dreidimensionalen Darstellen von Ultraschall-Bildern, insbesondere eines Körperorgans, vorgeschlagen (**deutsche Anmeldung P 39 08 648.8**), die einen Ultraschall-Scanner zur Aufnahme einer Schar von Schnittbildern und einen Rechner enthält, der aus der Stellung des Ultraschall-Transducers und der Lage der Pixel im Schnittbild dreidimensionale Bilder (perspektivische Projektionen) oder Bilder aus beliebigen Schnittebenen erzeugen und auf einem Bildschirm darstellen kann. Bei dieser Vorrichtung ist der Ultraschall-Scanner an einem mehrgliedrigen Arm am Gerät befestigt. Die Stellung der Arme und der Gelenke wird registriert und mit den aufgenommenen Bildern verknüpft. Der Führungsarm beschränkt die Handhabungsfreiheit des Arztes.

Auch ist es damit nicht einfach, eine koordinierte Schnittbildfolge zu erreichen, bei der die Ultraschallschnitte räumlich in einem fest definierten Verhältnis zueinander stehen. Dies ist aber die Voraussetzung für eine gute dreidimensionale Rekonstruktion. Beim Bewegen des Schallkopfes über die unebene Körperoberfläche ändert sich ständig der Einfallswinkel der Schallwellen. Eventuell wird auch das aufzunehmende Organ gedrückt oder verschoben. Eine Aufnahme paralleler Schnitte analog CT und MR erscheint daher für den klinischen Einsatz der dreidimensionalen Ultraschalldarstellung weniger geeignet zu sein.

Aufgabe der Erfindung ist es, eine solche Vorrichtung dahingehend zu verbessern, daß die Bildaufnahme möglichst frei und einfach und in kurzer Zeit erfolgen kann.

Diese Aufgabe wird erfindungsgemäß gelöst von einer Vorrichtung mit den Merkmalen des Anspruchs 1. Ausführungen der Erfindung sind Gegenstände von Unteransprüchen.

Günstig ist die relativ kleine und ortsfeste Ankoppelfläche des speziell dafür konstruierten Gehäuses, wodurch Unebenheiten der Körperoberfläche keinen störenden Einfluß ausüben.

Die erfindungsgemäße Anordnung eines Transducer-Arrays, insbesondere eines curved linear arrays, in einem handhabbaren Gehäuse und die motorische Bewegung erlauben dem Arzt eine frei wählbare Handhabung des Ultraschall-Scanners, da die Einsatzlage von keinem Führungsarm begrenzt ist. Bei der Bildaufnahme können sowohl das Eintrittsfenster als auch die Richtung praktisch vollkommen frei gewählt werden.

In einer Ausführung ist im Ultraschall-Scanner das Transducer-Array so integriert, daß es - von einer Schrittmotor-Steuerung und einem Schrittmotor angesteuert - eine Kreisbewegung oder eine Kippbewegung um eine Achse macht, die in der Schnittbildebene liegt und senkrecht zur Hauptabstrahlrichtung der Transducer verläuft. In einer bevorzugten Ausführung macht der Transducer eine Kippbewegung um maximal ± 30° aus der Mittelstellung. Der Radius der Kreisbewegung kann dem Radius des integrierten curved linear arrays angepasst sein. Diese Anordnung erlaubt eine kleine Ankoppelfläche. Die Aufnahme redundanter Daten, wie sie um die Mittelachse gedrehten Schallköpfen zu eigen ist, wird vermieden. In allen drei Raumachsen des dreidimensionalen Objekts kann die Auflösung des zweidimensionalen Ultraschallbilds in der entsprechenden Tiefe (Abstand zum Array) erreicht werden.

Die erfindungsgemäße transparente Darstellung der Projektion erzeugt - wie ein Phantombild in der Technik - einen klaren Eindruck vom Organ, insbesondere, wenn das Organbild in der Darstellung rotiert. Diese Darstellung kommt ohne die bisherigen Techniken der manuellen Konturierung, der Gewebedifferenzierung oder der Oberflächenrekonstruktion aus.

Die Erfindung wird anhand von vier Figuren näher erläutert.

Es zeigen:

Fig. 1 ein Blockschaltbild einer erfindungsgemäßen Vorrichtung,

Fig. 2 den Aufnahmebereich eines erfindungsgemäßen Schallkopfs,

Fig. 3 eine Ausführung eines erfindungsgemäßen Schallkopfs,

Fig. 4 eine Darstellungsart.

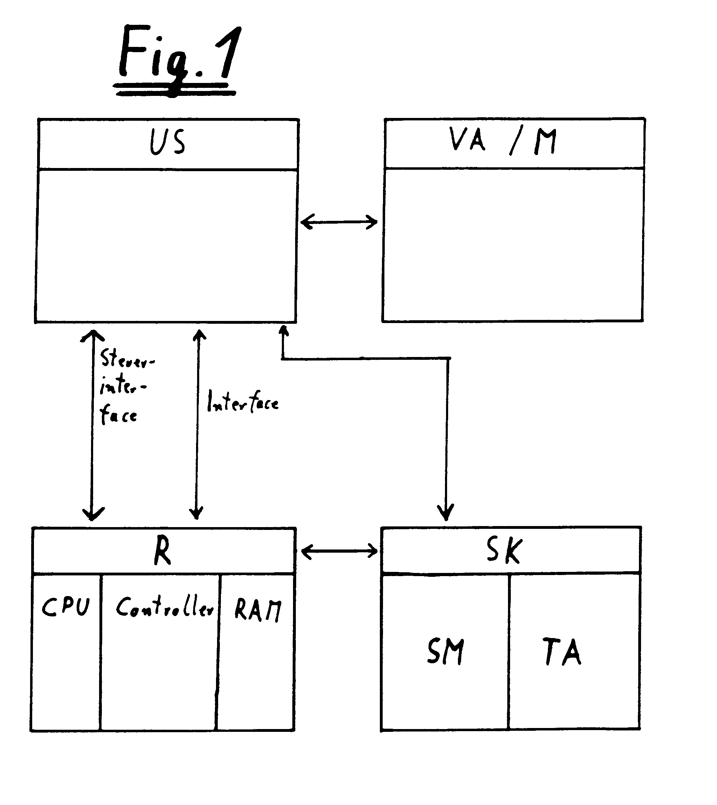

Fig. 1 zeigt im Blockdiagramm eines erfindungsgemäßen Geräts ein Ultraschallgerät **US** zur Erzeugung von Schnittbildern oder B-Bildern. Als visuelle Ausgabeeinheit **VA** mit Monitor **M** ist eine Anlage vorgesehen, die die Schnittbilder während der Diagnose und der Bildaufnahme anzeigen kann, die die dreidimensionalen Projektionen darstellen kann und die zur Darstellung frei definierbarer Schnittebenen aus dem dreidimensionalen Volumen verwendbar ist. Über ein Steuerinterface und/oder Interface für die Übertragung der Bilddaten ist das Ultraschallgerät **US** mit dem Rechner **R**

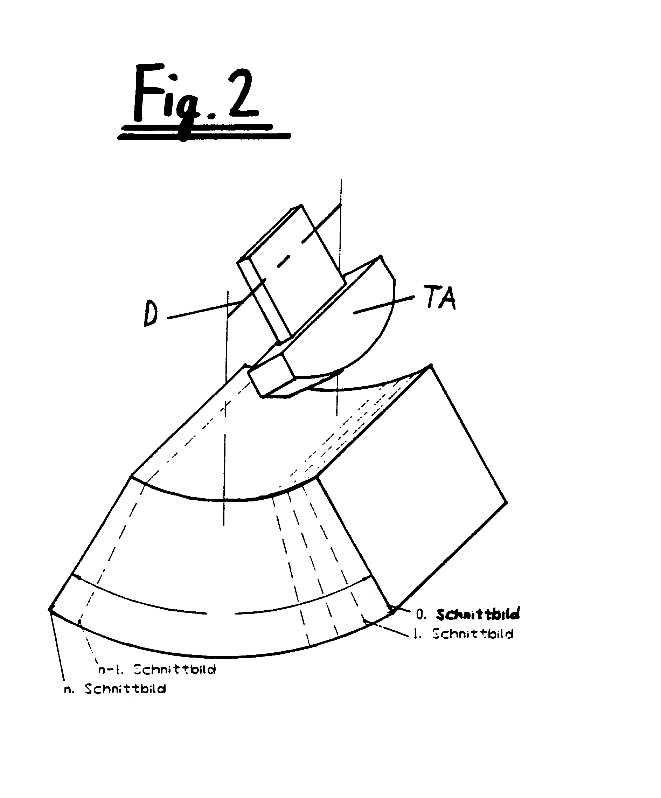

und dem Schallkopf **SK** verbunden. Der Rechner **R** erhält im wesentlichen die Zentraleinheit **CPU**, den Controller für den Schrittmotor und einen Speicher (**RAM**). Der Schallkopf **SK** enthält im wesentlichen den Schrittmotor **SM** und das Transducer-Array **TA**, das z.B. aus einer linearen Anordnung von einzelnen Schallsendern/Empfängern besteht, die auf einem Kreisbogen angeordnet sind (linear curved transducer array, Fig. 2).

Die Ultraschallschnittbilder können von einem Standard-Ultraschallgerät (z.B. Dornier Al 2200 HD) im 2D-Mode erzeugt werden. Als Rechner kann z.B. ein Compaq 386-Rechner mit 16 MByte RAM, benutzt werden. Das Bildverarbeitungssystem ist beispielsweise digital. Auch der Schrittmotorcontroller für die Positionierung des Transducers **TA** im Schallkopf **SK** ist handelsüblich. Der 3D-Schallkopf **SK** besteht im wesentlichen aus einem curved linearen Schallkopfarray, das um maximal ± 30° aus seiner Mittelstellung geschwenkt werden kann und über einen Schrittmotor angetrieben und positioniert wird. Mit diese Schallkopf **SK** ist es möglich, einen Volumenscan durchzuführen. Am Monitor **M** werden sowohl die einzelnen Schnittbilder während der Diagnose und Bildaufnahme als auch die Projektionen des rekonstruierten 3D-Objektes sowie frei wählbare, also auch sonographisch nicht erzielbare Schnitte durch das 3D-Objekt dargestellt.

Fig. 2 zeigt ein Transducer-Array **TA** und den Aufnahmebereich bei Verkippen oder Verschwenken (Verdrehen) des Transducer-Arrays **TA** um den maximalen Winkel 2 x 30° um eine Achse, die in der Schnittbildebene liegt und senkrecht zur Hauptabstrahlrichtung des Transducer-Arrays **TA** verläuft. Die Breite und die Tiefe des Bereichs, innerhalb dessen Organe liegen, die abgebildet werden können, entsprechen der Bildbreite und der Bildtiefe der zweidimensional gescannten B-Bilder.

Als Transducer im 3D-Schallkopf **SK** kommt hier z.B. ein curved linear array der Frequenz 3,5 MHz zum Einsatz. Das Transducer-Array **TA** ist derartig im 3D-Schallkopf **SK** gelagert, daß die Transduceroberfläche bei der Durchführung des Schwenks eine Kreisbahn beschreibt. Der Radius wurde dabei so gewählt, daß im Nahbereich des Transducers eine redundante Datenaufnahme vermieden und im Fernbereich eine für die angestrebten klinischen Anwendungen optimale Ausdehnung und Auflösung des Volumenscans erzielt wird.

Das dem verwendeten 3D-Schallkopf **SK** zugrundeliegende Prinzip des Schwenkens über einen einstellbaren Winkelbereich wurde gewählt, um die Aufnahme redundanter Daten zu vermeiden, wie sie bei Schallköpfen auftritt, die bei der Datenaufnahme um eine zentrale Bildachse rotieren. Neben dem für die Datenaufnahme in den Grenzen von hier ± 30° zur Mittelstellung wählbaren Schwenkwinkelbereich kann auch die Anzahl der in dem vorgegebenen Schwenkbereich aufzunehmenden 2D-Schnittbilder frei gewählt werden. Auf diese Weise kann die resultierende Auflösung des geschallten Volumens den klinischen Erfordernissen angepasst werden. Sowohl die Größe des gewählten Schwenkbereichs als auch die Anzahl der abzuspeichernden Schnittbilder in diesem Schwenkbereich bestimmen den für die Datenaufnahme eines Volumenscans aufzuwendenden Zeitraum. Der zur Datenaquisition benötigte Zeitraum betrug bei einem Prototyp je nach Anwendung ca. 15 bis 30 Sekunden, was eine bequeme und fehlerfreie Aufnahme z.B. im Abdominalbereich erlaubt, wo keine schnellen zeitlichen Änderungen des geschallten Bereichs zu erwarten sind.

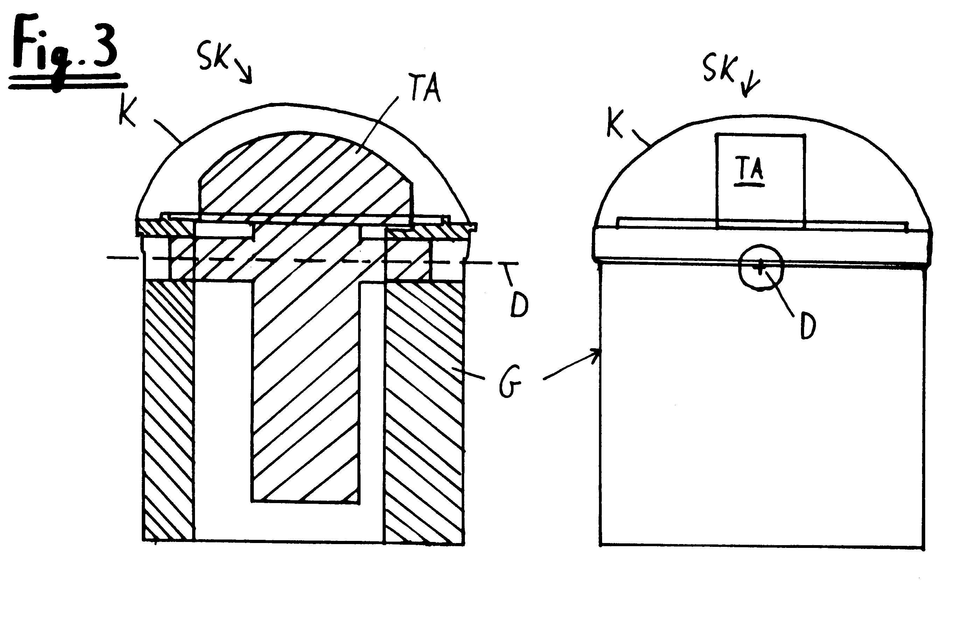

Fig. 3 zeigt eine Ausführung eines erfindungsgemäßen Schallkopfes **SK** einmal im Schnitt und einmal dagegen gedreht in Draufsicht. In dem Gehäuse **G** das Transducer-Array **TA** angebracht ist und wird von einem Schrittmotor **SM** (nicht gezeigt) gedreht. Eingezeichnet ist die Drehachse **D** für das Verdrehen des Transducer-Arrays **TA**. Die Schallkopfkappe **K** dient zur Ankopplung des Schallkopfs **SK** an den Patientenkörper.

Die Vorrichtung funktioniert wie folgt:

## Bildaufnahme

Über eine (z.B. interaktive) Bedienerführung kann der Benutzer die Größe des aufzuzeichnenden Volumenelements im Körper eingeben und die eingestellten Werte durch Testscans mit den gewählten Parametern überprüfen. Der Benutzer bestimmt ebenfalls die Anzahl der Schnittbilder, die für den Volumenscan aufgenommen werden sollen. Die Testscans und die Aufnahme der Schnittbilder können über einen Fußschalter gestartet werden, so daß der Arzt beide Hände zur Führung und Fixierung des Schallkopfes **SK** am Patientenkörper während des Volumenscans nutzen kann. Während der Bildaufnahme sollte der Schallkopf keine Relativbewegung gegenüber dem Patienten durchführen, da sonst die einzelnen Schnittbilder falsch zugeordnet werden.

Zur Bildaufnahme wird das Transducer-Array **TA** mit Hilfe des Schrittmotorcontrollers im Rechner **R** und des Schrittmotors **SM** positioniert und das entsprechende digitalisierte Schnittbild inklusive der Position des Transducer-Arrays **TA** abgespeichert (Speicher **RAM**). Der Zugriff auf das Schnittbild kann über eine digitale Schnittstelle zum Bildspeicher des Ultraschallgeräts **US** oder über die Digitalisierung des Videosignals des Ultraschallgerätes **US** erfolgen. Sobald ein Schnittbild aus dem Bildspeicher des Ultraschallgeräts **US** übernommen wurde, wird das Array **TA** neu positioniert. Die Schrittweite ergibt sich aus der Größe des Volumenscans und der geforderten Anzahl von Schnittbildern, damit ergibt sich auch die erreichbare Auflösung. Die Ausgestaltung der Ankoppelfläche **K** des 3D-Schallkopfes **SK** hat bevorzugt die Form einer Kugelfläche. Dies ist anatomisch günstig bei verhältnismäßig geringer Ausdehnung. Durch die gewählte Genauigkeit des zur Arraypositionierung verwendeten Schrittmotors können je Winkelgrad des geschallten Volumenscans bis zu drei aufeinanderfolgende 2D-Schnittbilder aufgenommen werden. Nach jeder neuen Positionierung des Transducer-Arrays **TA** werden die vom Ultraschallgerät **US** erzeugten Bilddaten digital abgespeichert (Rohdaten) und in dieser Form der darauffolgenden Bearbeitung zugeführt. Durch Transformation jedes einzelnen Bildpunktes aller aufgenommenen Schnittbilder in ein kartesisches dreidimensionales Koordinatensystem wird die Ultraschallinformation des geschallten Volumenbereichs rekonstruiert. Dabei bestimmen die Koordinaten des einzelnen Pixels im zweidimensionalen Schnittbild sowie die Lage des Schnittbildes im aufgenommenen Winkelbereich die Positionszuordnung im dreidimensionalen Koordinatensystem. Als Ergebnis erhält man die räumlich korrekte Zuordnung aller aufgenommenen Ultraschallinformationen (dreidimensionaler Rohdatenspeicher).

Einschließlich dieses Schrittes hat noch keinerlei Datenreduktion oder Veränderung der ursprünglichen Ultraschallinformation durch Nachverarbeitung stattgefunden (keine Informationsverluste).

**Dreidimensionale Rekonstruktion**

Zur Rekonstruktion des dreidimensionalen Objektes werden die Bildpunkte der zweidimensionalen Schnittbilder in einen dreidimensionalen Bildspeicher transformiert. Die Koordinaten der Pixel errechnen sich aus ihrer Position im Schnittbild und der jeweiligen Stellung des Transducer-Arrays **TA**. Die Auflösung im dreidimensionalen Bildspeicher kann für alle drei Raumachsen vom Benutzer eingestellt werden.

**Dreidimensionale Darstellung**

Für die Darstellung des dreidimensionalen Objekts ergeben sich zwei Möglichkeiten:

a) Zweidimensionale Darstellung beliebiger Schnitte durch das dreidimensionale Objekt, insbesondere durch das geschallte Körperorgan. Durch eine interaktive Benutzerführung kann die Lage der Schnittebene frei definiert werden, das heißt, daß auch Schnitte rekonstruiert werden können, die mittels Ultraschall sonographisch nicht aufgenommen werden können. Weiter kann das Objekt, mittels mehrerer paralleler, aufeinanderfolgender Schnitte, in beliebiger Richtung durchgeblättert (analysiert) werden.

Die Schrittweite und die Orientierung können wieder vom Benutzer eingegeben werden.

b) Perspektivische Projektion des Objekts auf die Bildebene des Monitor **M**.

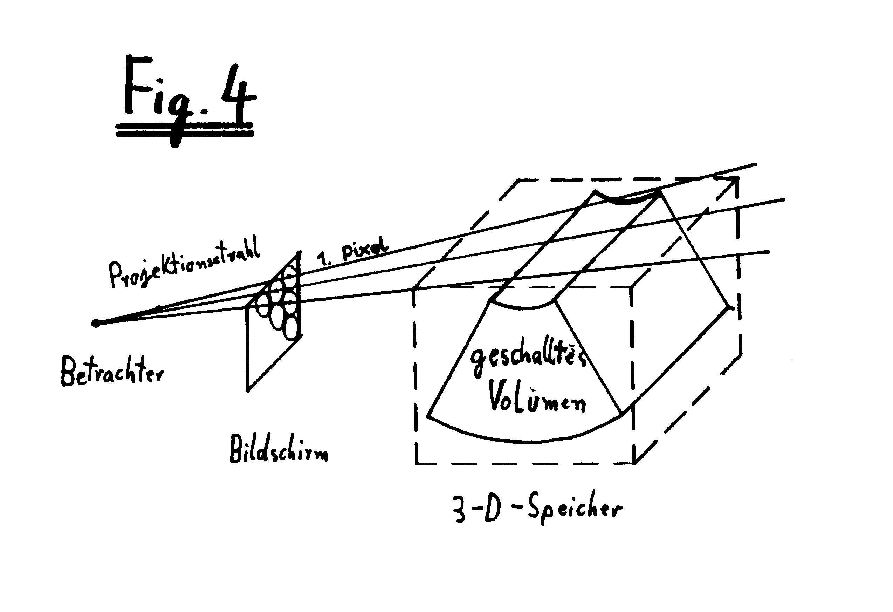

Für viele Anwendungen kann jedoch die räumliche Darstellung der gesamten dreidimensionalen Information einen wesentlich höheren diagnostischen Informationsgehalt vermitteln. Die räumliche Darstellung in der Bildebene (Monitor **M**) erfolgt durch perspektivische Projektion aller Bildpunkte des dreidimensionalen Rohdatenspeichers. Mit dem anhand von Fig. 4 beschriebenen Algorithmus wird der gesamte rekonstruierte Raum transparent dargestellt. Alle Projektionsstrahlen treffen sich im Betrachterstandpunkt, schneiden die Projektionsebene (Bildebene oder Bildschirmebene) und verlaufen durch das darzustellende Objekt. Die Anzahl der Projektionsstrahlen wird durch die Anzahl der Pixel in der vom Benutzer gewählten Bildgröße auf dem Monitor **M** bestimmt. Der Grauwert eines Bildpunktes am Monitor **M** wird errechnet aus der Summe aller auf dem Projektionsstrahl liegenden Objektpunkte. Der resultierende Grauwert des Objektpunktes errechnet sich in Abhängigkeit vom Abstand zum Betrachter, seiner Umgebung auf dem Projektionsstrahl und seinem ursprünglichen Grauwert.

Die dreidimensionale Wahrnehmung der Darstellung der Projektionsebene kann verstärkt werden, indem die räumlichen Grenzen des durchgeführten Volumenscans angedeutet werden.

Die Koordinaten des Betrachters können frei gewährt werden. Das Objekt wird transparent dargestellt, indem alle Pixel, die auf einem Projektionsstrahl liegen, bewertet und aufaddiert werden. Die Bewertung erfolgt in Abhängigkeit vom Abstand des Pixels zum Betrachter, vom Grauwert des Bildpunktes und seiner Umgebung. Zusätzlich kann eine Bewertung der Pixel aufgrund externer Beleuchtung des dreidimensionalen Objektes nützlich sein.

Das dreidimensionale Volumenelement kann von beliebigen Betrachterstandpunkten mittels orthogonaler oder perspektivischer Projektion auf die Bildebene des Monitors **M** projeziert und mit variabler Transparenz dargestellt werden. Diese Darstellung erlaubt im Gegensatz zu den bekannten zweidimensionalen Ultraschall-Schnittbildern die Beurteilung und Definition der räumlichen Ausdehnung und Lage von Organen und Strukturen. Außerdem können Schnittebenen durch das dreidimensionale Volumen frei definiert und rekonstruiert werden. Damit können Schnittbilder dargestellt werden, die wegen der unerreichbaren Ausgangslage für den Schallkopf sonographisch normalerweise nicht gewonnen werden können.

Die Transparenz und der dreidimensionale Eindruck des Objekts werden deutlich verbessert durch eine zeitlich koordinierte aufeinanderfolgende Darstellung von Projektionen aus unterschiedlichen Betrachterstandpunkten. Wird z.B. eine Kreisbewegung des Betrachterstandpunktes um das Objekt vorgenommen, so führt dies zu einer Rotation des Objekts am Bildschirm. Für den Betrachter scheint sich ein durchsichtiges dreidimensionales Modell des Organs am Bildschirm langsam zu drehen. Es entsteht der Eindruck eines im Raum rotierenden, dreidimensionalen Objekts (dreidimensionale Animation). Dieser Effekt wird erzielt, indem man den Betrachterstandpunkt z.B. in einer frei zu wählenden Kreisbahn um das darzustellende Objekt verändert, die zugehörigen Projektionen berechnet und und zur Darstellung bringt.

Lage und Ausdehnung dreidimensionaler Objekte können auf diese Weise leicht erkannt und analysiert werden. Mittels dieser Darstellungstechnik werden vor allem Transparenz und dreidimensionaler Charakter bei der Wahrnehmung betont. Es ergeben sich weit über die einfache Projektion dreidimensionaler Daten hinausgehende Analyse- und Auswertemöglichkeiten.

**Patentansprüche**

1. Vorrichtung zum dreidimensionalen Darstellen von Ultraschallbildern, insbesondere eines Körperorgans, mit
   - einem Ultraschallgerät (**US**) zur Aufnahme einer Schar von Schnittbildern und
   - einem Rechner (**R**), der aus der Stellung des Transducer-Arrays (**TA**) und der Lage der Pixel im Schnittbild dreidimensionale Bilder oder Bilder aus beliebigen Schnittebenen erzeugt und auf einen Bildschirm (Monitor **M**) darstellt,
   **dadurch gekennzeichnet**, daß der Schallkopf (**SK**) in einem handhabbaren Gehäuse (**G**) angeordnet ist, ein lineares Transducer-Array (**TA**) und einen Motor (Schrittmotor **SM**) enthält, der das Transducer-Array (**TA**) im vorgegebenen Winkelbereich positioniert, wobei die jeweiligen Stellungen des Transducer-Arrays (**TA**) registriert werden, und daß die dreidimensionalen Bilder transparente perspektivische Projektionen sind.

2. Vorrichtung nach Anspruch 1, **dadurch gekennzeichnet**, daß das Transducer-Array (**TA**) um eine Achse (**D**) verdreht wird, die in der Schnittbildebene und senkrecht zur Hauptabstrahlrichtung liegt.

3. Vorrichtung nach Anspruch 2, **dadurch gekennzeichnet**, daß das Transducer-Array (**TA**) periodisch um maximal 2 x 30° verkippt wird.

4. Vorrichtung nach einem der vorhergehenden Ansprüche, **dadurch gekennzeichne**t, daß ein lineares Transducer-Array (**TA**), insbesondere ein curved linear Transducer-Array (TA) vorgesehen ist.

5. Vorrichtung nach Anspruch 1, **dadurch gekennzeichnet**, daß Projektionen aus unterschiedlichen Betrachtungsstandpunkten zeitlich nacheinander folgend dargestellt werden, so daß eine Bewegung, insbesondere eine Rotation, des Abbilds des Organs stattfindet.

# Fig.1

| US | VA / M |
|---|---|

Steuerinterface

Interface

| R | SK |
|---|---|
| CPU | Controller | RAM | SM | TA |

# Fig. 2

D — TA

0. Schnittbild
1. Schnittbild
n-1. Schnittbild
n. Schnittbild

Fig.3

Fig. 4

Projektionsstrahl

1. Pixel

Betrachter

Bildschirm

geschalltes Volumen

3-D-Speicher

EP 0 476 495 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-2 027 197 (DIASONICS, INC.) <br> * Seite 1, Zeilen 50-55; Seite 2, Zeile 28 - Seite 3, Zeile 19; Seite 5, Zeilen 4-46; Figuren 1,2,3a,3b,10 * <br> – – – | 1-3 | A 61 B 8/14 <br> G 10 K 11/00 <br> G 01 S 7/52 |
| X | EP-A-0 037 012 (SIEMENS AG) <br> * Seite 3, Zeile 20 - Seite 4, Zeile 37; Figuren 1,2 * <br> – – – | 1,2,4 | |
| X | US-A-4 932 414 (D.J. COLEMAN et al.) <br> * Spalte 4, Zeile 48 - Spalte 6, Zeile 7; Figur 2 * <br> – – – | 1,2 | |
| X | IEEE COMPUTER SOCIETY, COMPUTERS IN CARDIOLO-GY 25.-28. September 1988, Seiten 21-26, Washington, D.C., US; R.H. SELZER et al.: "Computer-generated 3D ultrasound images of the carotid artery" <br> * Seite 21, Zusammenfassung; Seite 22, linke Spalte, Zeile 24 - Seite 24, linke Spalte, Zeile 35; Figuren 1-3 * <br> – – – | 1,2 | |
| P,X | EP-A-0 432 771 (ALOKA CO. LTD.) <br> * Spalte 4, Zeile 3 - Spalte 6, Zeile 49; Figuren 1-4 * <br> – – – – – | 1,2,4 | |
| | | | **RECHERCHIERTE SACHGEBIETE (Int. Cl.5)** <br><br> A 61 B 8/00 <br> G 10 K 11/00 <br> G 01 S 7/00 |

Der vorliegende Recherchenbericht wurde für alle Patentansprüche erstellt

| Recherchenort | Abschlußdatum der Recherche | Prüfer |
|---|---|---|
| Berlin | 11 Dezember 91 | WEIHS J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

------------------------------------------------------------

& : Mitglied der gleichen Patentfamilie, übereinstimmendes Dokument